Europäisches Patentamt

European Patent Office

Office européen des brevets

(11) Publication number: **0 108 527**
**A2**

# EUROPEAN PATENT APPLICATION

(21) Application number: **83306306.8**

(22) Date of filing: **18.10.83**

(51) Int. Cl.³: **G 01 N 21/25**
**G 01 N 21/05, G 02 B 5/16**

(30) Priority: **03.11.82 US 438644**

(43) Date of publication of application:
**16.05.84 Bulletin 84/20**

(84) Designated Contracting States:
**DE FR GB IT NL**

(71) Applicant: **INDIANA UNIVERSITY FOUNDATION**
**Showalter House P.O. Box 500**
**Bloomington Indiana 47401(US)**

(72) Inventor: **Brown, Daniel J.**
**441 Halsted Court**
**Indianapolis Indiana 46224(US)**

(74) Representative: **Massey, Alexander et al,**
**MARKS & CLERK Scottish Life House Bridge Street**
**Manchester, M3 3DP(GB)**

(54) Remote optic atmosphere monitor and method for determining a foreign substance in a fluid.

(57) A device and method for determination of a foreign substance in a fluid. A fiber optic cord is wound back and forth on a plurality of upstanding posts encapsulated within a solid mass. A plurality of openings extend through the mass transecting the cord at multiple locations along the cord path. A specified light is directed through the cord with the fluid and foreign substance being introduced into the openings. The light exiting the cord is measured with the foreign substance then being determined. An alternate embodiment includes a fiber optic cord enclosed within a membrane with a plurality of openings being provided in the cord. The cord is percutaneously injected into a person while attached to a light source and detector apparatus allowing for the determination of the foreign substance in the fluid being measured.

EP 0 108 527 A2

1

# REMOTE OPTIC ATMOSPHERE MONITOR AND METHOD
# FOR DETERMINING A FOREIGN SUBSTANCE IN A FLUID

## Background of the Invention

Field of the Invention: The present invention relates to the sample chamber, cuvettes or sample cells used in most infrared, ultraviolet or visible wavelength spectrophotometer instruments designed to detect, measure or quantify specific chemical substances.

Description of the Prior Art: Spectrophotometric instrumentation, used for the qualitative and quantitative determination of chemical substances, plays a principle role in the disciplines of medicine, chemistry and physics, as well as in military and industrial applications and a variety of other uses. These instruments have a function based on a phenomenon of nature in which atoms and/or molecules either emit or absorb energy as they change from one energy state to another. The type of energy involved in this process is electromagnetic radiation, that is, light (visible, infrared, ultraviolet), X-rays, radio and radar waves. These are all simply different parts of a broad spectrum that stretches from gamma rays, whose wavelengths are measured in fractions of Angstrom units, to radio waves, whose wavelengths are measured in meters or even kilometers.

When a beam of electromagnetic radiation is passed through a substance, the radiation (energy) can be either absorbed or transmitted, depending upon its frequency and the structure of the molecules it encounters. The energy gained by the molecule in this way may bring about increased vibration or rotation of the atoms, or may raise electrons to higher energy levels. The particular frequency of radiation that a given molecule can absorb depends upon the changes in vibrations or rotation or electron states that are permitted to a molecule of that structure. Each chemical compound, therefore, has a spectrum that is a plot that shows how much electromagnetic radiation is absorbed or transmitted at each frequency. This spectrum is highly characteristic, like a finger print of the chemical compound's structure.

Spectrophotometric instruments of the prior art generally use light as the energy medium for chemical detection. Light of wavelengths between about 4,000 Angstroms and 7,500 Angstroms is visible. Just beyond the red end of the visible spectrum (wavelengths greater than 7,500 Angstroms) lies the infrared region of light. Just beyond the violet end of the visible spectrum (wavelengths of less than 4,000 Angstroms) lies the ultraviolet region of light.

Ultraviolet spectrophotometers commonly used, measure the absorption of light in the visible and "near" ultraviolet region, that is, in the 2,000 to 7,500 Angstrom range. This light is of higher frequency and greater energy than infrared light and when it is absorbed by a molecule, the changes it produces are generally ones that require greater energy, such as changes in electron states.

In a transition to a higher electron level, a molecule can go from any of a number of sub-levels, corresponding to various vibrational and rotational states, to any of a number of other sub-levels. As a result, ultraviolet light absorption bands are broad, and a typical ultraviolet spectrum shows only a few broad humps. Such a spectrum can conveniently be described in terms of the position of the top of the hump (wavelength maximum) and the intensity of that absorption (the extinction coefficient). Thus, the ultraviolet spectrum is used primarily to demonstrate relationships between functional groups (chiefly conjugation) rather than to show the presence of individual functional groups. This, however, provides adequate information for the qualitative and quantitative identification of chemical compounds.

Of all of the properties of a chemical compound, the one that, by itself, gives perhaps the most information about the chemical's structure, is its infrared spectrum. The infrared spectrum helps to reveal the structure of a compound by defining what constituent groups are present in, or absent from, the molecule. A particular group of atoms absorbs light of certain frequencies that are much the same from compound to compound, that is to say, they give rise to characteristic absorption bands. For example, the hydroxyl group (-OH) of alcohols absorbs strongly at 28,000 to 33,000 Angstroms and the methyl group (-CH$_3$) absorbs strongly at 68,000 to 75,000 Angstroms. Therefore, two substances that have identical infrared spectra are, in effect, identical in thousands of different physical properties and must certainly be the same compound.

Interpretation of an infrared spectrum is not a simple matter, but absorption at even a few wavelengths may provide adequate information for qualitative and quantitative identification of chemical compounds.

Spectrophotometric instrumentation of the prior art is generally constructed such that light from a broad spectrum source (conventional incandescent bulb) is separated via prisms or a diffraction grating into discrete wavelengths. The monochromatic light can then be focused and collimated by lenses and mirrors so that the light is passed through some sort of sample chamber where high transparency sample holders (cuvettes or sample cells) containing the chemical substance being analyzed intercept the energy beam. The light passes through the chemical substance and continues on, via lenses and mirrors, to a detector apparatus, such as a photomultiplier tube. The signal generated by the detector apparatus is then amplified electronically and displayed or recorded. By altering the angle of the prisms or the diffraction grating, and by widening or narrowing light path slits, a continuous spectrum of light is passed through the sample. Specific wavelengths absorbed by the chemical substance as it changes energy states are noted by their absence in the recording. Interference from absorbance of the sample holder or any other extraneous source can be factored out by a comparison of the sample beam of light with a reference beam of the same light passed through the same course except for the presence of the chemical substance being analyzed.

Some of the limitations of the usefulness of spectrophotometric instruments has been brought about by the sample cell or cuvette that holds the chemical sample. Since the sample cell must intersect the energy beam of the instrument, the cell has been fixed within the instrument, and this necessitates bringing the chemical sample to the instrument after suitable collection effort and preparation. The size of the sample cell is also limited (typically to a one-centimeter path length) by the space it can occupy within the confines of the instrument, and this can effectively reduce the sensitivity of the analytical determination, especially for gases and vapors. These particular limitations are overcome with

5

the present invention by using fiber optic light-wave guides as both the means of carrying the light energy significant distances from the instrument to the chemical sample, and as the sample cell itself, which can have unusually large dimensions.

A traditional approach of determining alcohol content of blood is shown in U.S. Patents 4,057,724, issued to Adrian et al. and 4,090,078, issued to Heim. In these patents, the subject's breath is introduced into a sample chamber through which infrared energy is passed to an infrared detector or measuring instrument, providing a readout of the alcohol content. A further gas analyzer utilizing infrared radiation is disclosed in U.S. Patent 4,180,734, issued to Gedeon and the U.S. Patent 3,562,524, issued to Moore et al. In spite of the above advances, there is yet no atmosphere monitor cell utilizing fiber optics to conveniently, accurately and efficiently direct a source of light through the fluid to be tested for a foreign substance. Disclosed herein is such a device.

0108527

6

## Summary of the Invention

One embodiment of the present invention is a device for testing a substance in a fluid comprising a frame, fiber optic means to conduct light being mounted to the frame and extending through a path, the means including at least one opening extending therein allowing the substance to flow therein to receive said substance and change the light directed through the means.

Another embodiment of the present invention is a device for checking a substance in a fluid comprising elongated fiber optic means including a plurality of openings extending therein changing the light directed through the means.

A further embodiment of the present invention is an optical test cell to be used with a light measuring instrument comprising a fiber optic cord having a plurality of openings into which the substance to be tested may flow.

Yet another embodiment of the present invention is a remote optical atmosphere monitor cell comprising a plate with upstanding posts, a fiber optic element wound around the posts and extending therebetween in multiple layers, and, a solidified mass enclosing the element and having a plurality of slots extending across and through the element into which the fluid to be monitored may flow.

A further embodiment of the present invention is a method of detecting a foreign substance in a fluid comprising the steps of extending a fiber optic cord through a path, cutting openings in the cord at multiple locations between its opposite ends, directing the fluid into the opening, directing light through the cord; and comparing the light directed through the cord with the fluid in the openings to a standard.

It is an object of the present invention to provide a device for checking a substance in a fluid.

7

Yet a further object of the present invention is to provide a new and improved remote optical atmosphere monitor cell.

In addition, it is an object of the present invention to provide a new and improved method for detecting a foreign substance in a fluid.

Related objects and advantages of the present invention will become apparent from the following description.

8

## Brief Description of the Drawings

FIG. 1 is a block diagram of the remote optical atmosphere cell (ROAM) connected to a spectrophotometer.

FIG. 2 is a plan view of the ROAM Cell shown in FIG. 1.

FIG. 3 is a cross-sectional view taken along the line 3-3 of FIG. 2 and viewed in the direction of the arrows.

FIG. 4 is an end view of the ROAM Cell of FIG. 2.

FIG. 5 is the same view as FIG. 2 only showing an alternate embodiment of the ROAM Cell.

FIG. 6 shows a fiber optic member, a portion of which is enlarged, for percutaneous injection to monitor substances present in blood.

## Description of the Preferred Embodiments

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

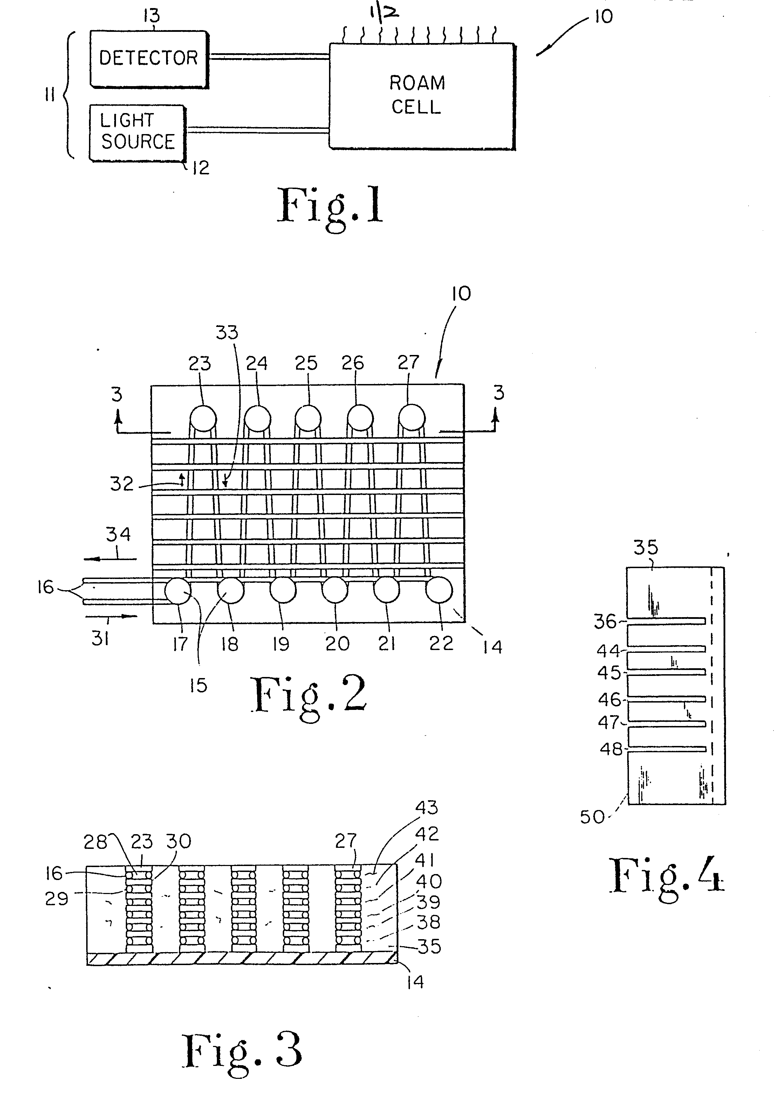

Referring now more particularly to the drawings and to FIG. 1, there is shown a device for testing a foreign substance in a fluid such as a gas. The device includes a remote optical atmosphere monitor (ROAM) cell 10 operably connected to a spectrophotometer 11 including a light source 12 directing light into cell 10 and a detector or comparator 13 for the determination of the amount of light exiting cell 10.

Cell 10 includes a mounting plate 14 (FIG. 2) upon which are fixed a plurality of winding posts 15 or similar structures in appropriate number and spacing to accommodate the anticipated length and size of a fiber optic light-wave guide 16. In the embodiment shown in FIG. 2, six winding posts are cantileveredly mounted along one longitudinally extending edge portion of plate 14 with five additional mounting posts being cantileveredly mounted to the opposite edge portion of the plate and interspaced between the six posts. Thus, posts 17-22 are mounted to one side of plate 14 with posts 23-27 mounted to the opposite side. The winding posts are positioned such that the inside of post 17 aligns with the outside of opposite post 23 which in turn is aligned in similar fashion with post 18 and with the remaining posts aligned in similar fashion.

Each mounting post is identical in configuration and has a series of circular grooves of specific size, number and spacing around its circumference such that the light guide fiber can fit in the groove to maintain alignment and separation. For example, post 23 has a top circumferentially extending groove 28 for receiving light guide 16 with the next lower groove 29 being separated by a plate 30 integral with post 23. Light guide 16 is of conventional fiber optic design being cord configured and wound around each mounting post in sequential fashion. Light guide 16 is first wound around the lower groove of the first mounting post 17 and thus is spaced above plate 14. Guide 16 is bonded to post 17 with a permanent bonding epoxy or similar adhesive. After the adhesive is cured, guide 16 is passed, at the proper tension to maintain a straight axis, to the outside of post 23 and back and forth between each post in a parallel fashion, resting in the bottom groove until reaching the inside bottom of the last post 22. At this point, the light guide 16 is wound around and crosses in front of post 22 and is passed back to the second groove from the bottom of post 17 to repeat the process until all the grooves are filled. The winding of guide 16 on the mounting post can be set to whatever level is required for a specific monitoring function, and when the last level is achieved, the guide is bonded with the permanent bonding adhesive to the top of post 17 exiting the cell in the direction of arrow 34. Thus, a specified amount of light is directed into the cell in the direction of arrow 31 and passes from post 17 to post 23 in the direction of arrow 32, from post 23 to post 18 in the direction of arrow 33 and back and forth until finally exiting the cell in the direction of arrow 34.

Once the light guide 16 is wound around the mounting posts, the guide and mounting posts are encased or encapsulated within an appropriate material such as a potting compound 35 (FIG. 3) including plastic, resin, etc. An open top embedding box may be used to form compound 35. Such a box is sized to receive plate 14 along with the mounting posts with two holes provided in the box of appropriate size to accommodate guide 16 entering and exiting the cell. The mounting plate 14 with the fiber wound mounting posts 15 are placed into the embedding box such that the entering and exiting wave guide 16 passes through the holes provided in the box. The embedding box is then filled with an appropriate material to embed the wave guide in a solid matrix 35 that is allowed to solidify completely in the configuration shown in FIG. 3. The solid matrix is initially in a flowable state and is poured into the box and subsequently allowed to solidify.

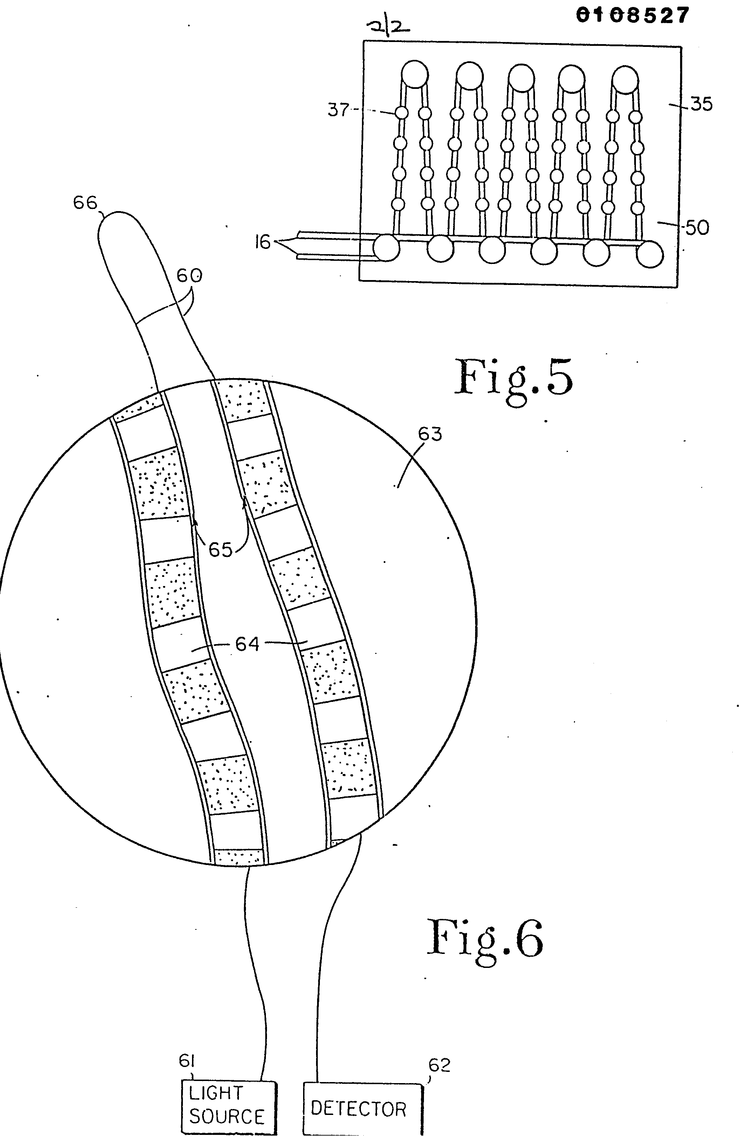

Following matrix solidification, a plurality of slots 36 (FIG. 4) of varying number and dimensions are cut or holes 37 (FIG. 5) are drilled perpendicular to the direction and plane of light guide 16. These slots 36 or holes 37 transect light guide 16 that passes from post to post and creates a gap that separates, or a hole that penetrates, the core of the light guide. Slots 36 and 44-48 are perpendicular to cord 16 as the cord extends back and forth from mounting post to mounting post. The spacing between the slots 36 or holes 37 is selected with consideration being given to optical coupling and spillover, phenomena associated with fiber optic light transmission from fiber to fiber, both of which are functions of the diameter of the fiber core and the numerical aperture of the optical fiber. Generally, this spacing amounts to only a fraction of the core diameter. As seen in FIGS. 4 and 5, by embedding the light guide 16 in the solid matrix 35, the light guide can be cut many times while allowing the fiber axis to remain perfectly aligned. Thus, maximum optical signal coupling across the slots or holes will be realized and will be limited only by spillover, which may be determined from the gap spacing and the numerical aperture of the light guide 16. In the example shown in FIG. 4, the solid matrix 35 is provided with a total of six slots all of which extend downwardly to slightly above plate 14 while cutting the light guide as it extends through a back and forth path between mounting posts. The light guide is wound around the posts to create six different horizontal layers which are cut by the six slots. That is, light guide 16 is arranged in six layers 38-43 (FIG. 3) with each layer being cut by the six slots 36 and 44-48 (FIG. 4).

Following the formation of the slots or holes in the solid matrix, the cut surfaces of light guide 16 are shaped, polished or finished to the appropriate degree in order to obtain maximum optical signal coupling. Commercially available devices, such as prisms, designed to gather or deliver light are fitted to the external ends of light guide 16 in turn attached to the light source 12 and detector apparatus 13.

The ROAM Cell contains no moving parts. It functions, by passing a beam of frequency-modulated, optically-filtered and collimated light of suitable wavelength from sources, such as lasers, conventional lamps or light emitting diodes. These light sources, with the appropriate modulating, filtering and collimating systems, exist in instruments designed to quantify, measure or detect chemical substances via the process of light absorption at specific wavelengths by specific chemical molecular structure. Instruments of this type generally contain fixed cells, chambers or cuvettes that are filled with the chemical of interest, and through which the light energy is passed in order to interact the light with the chemical substance. The ROAM Cell will substitute for or replace these fixed cells or chambers and function at a site remote to the instrument. In this invention, the fiber optic light-wave guide 16 carries the light source from the adapted commercial instrument to the cut surfaces of the slots or holes. A commercially available spectrophotometer which may be utilized with the ROAM Cell is Beckman Model 35, manufactured by Beckman Instrument, Inc., Irvine, California 92713. A spectrophotometer includes a light source of ultraviolet frequency from a Deuterium source such as Beckman Model No. 585699 or a visible light from a Tungsten source such as Beckman Model No. 56333. The optical signal propagating along the guide 16 will couple across the gap of the slot or hole and through the gap fluid media (gas or liquid) which contains the chemical substance to be quantified, measured or detected. The top surface 50 of the solid matrix is positioned to receive the incoming fluid with the foreign substance to be detected. Thus, in the case of detecting gases emitting from the skin of a person, surface 50 is placed in contact with the skin. Because the number of slots, or holes, the dimensions of the gaps, and the length and number of fiber layers are all variable, the path length of light through the slot or hole gap media can be any length practical. Therefore, light absorption in the gap by a specific chemical substance can provide a high degree of detection sensitivity, since this is a function of light path length.

After coupling through the initial slot 48 gap media, the light is carried through the guide 16 to the next slots 47-44 and 36, then around post 23 to recross the slots again. This process can be repeated hundreds of times and since the light path is constructed in series, any light absorption at any single slot gap crossing is additive with any light absorption at any other crossing.

After completing the slot crossings, the light returns via the exit fiber to the detector portion of the instrument being utilized and is appropriately quantified to determine the amount of light absorbed by the specific chemical substance. The amount of light absorbed by the specific chemical substance is directly related to the substance concentration and this concentration can easily be computed and displayed.

The method of detecting a foreign substance in a fluid includes the step of extending the fiber optic cord through a path and then cutting openings in the cord at multiple locations between its opposite ends. The fluid to be tested is directed into the openings with light passing through the cord, across the openings and through the fluid. The light directed through the cord, openings and fluid is then compared to a standard fluid without the foreign substance being present. As previously explained, certain foreign substances in fluids absorb specific wave-lengths of light energy. Thus, knowing the amount of electromagnetic energy at a specified wavelength directed initially into the ROAM Cell and by measuring the amount of light energy exiting the ROAM Cell at specific wavelengths, it may be easily determined the type and amount of foreign substance within the fluid being measured.

FIG. 5 shows another embodiment of the invention which may be utilized to quantify chemical substances in small spaces, such as blood vessels of the human body. The core of the optical fiber 60 (shown greatly enlarged at location 63) is pierced by laser drill or chemical etching, to produce a series of spaces or gaps 64 along its length, that are perpendicular to the axis of the fiber 60 core. The fiber 16 is then coated with a semi-permeable membrane 65 that allows the passage of water and water soluble chemicals into the gaps 64. Such a semi-permeable membrane is commercially available from Spectrum Medical Industries, Inc., 60916 Terminal Annex, Los Angeles, California 90054 and is a natural cellulose membrane tubing. Specific light wavelengths from the instrumentation source 61 pass through the fiber 60, cross

15

the gaps 64, and are absorbed by the chemical present in the aqueous media that infiltrates from the blood, through the membrane 65 and into the gaps 64. The absorption of the light is noted by the detector apparatus 62 and the concentration of the chemical substance computed and displayed. The fiber 60 is small enough to fit through a hypodermic needle device that allowing continuous monitoring of blood concentrations of chemicals. Fiber 60 is sized to be injected percutaneously into a person with the outer end 66 being folded over as shown in the drawing and injected into the person with the opposite ends being attached to the light source and detector.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

16

WHAT IS CLAIMED IS:

1.  A device for testing a substance in a fluid comprising:

a frame;

fiber optic means to conduct light being mounted to said frame and extending through a path, said means including at least one opening extending therein allowing said substance to flow therein and change said light directed through said means.

2.  The device of claim 1 and further comprising second means mounted to said frame and including a channel in communication with said opening allowing said substance to flow therethrough, said first means includes a fiber optic member.

3.  The device of claim 2 and further comprising light source means connected to one end of said member and operable to apply a predetermined quantity of light to said one end; and,

light detection means connected to an opposite end of said member and operable to measure said light exiting said fiber optic member.

4.  The device of claim 3 wherein said second means includes a plurality of slots formed therein which extend transversely through and interrupting said member at multiple locations allowing said substance to be disposed across and through said member.

5. A device for checking a substance in a fluid comprising:

elongated fiber optic means including a plurality of openings extending therein to receive said substance changing the light directed through said means.

6. The device of claim 5 wherein said means includes a fiber optic member with a plurality of holes extending transverse to said member and a membrane extending around said member allowing said substance to flow therethrough into said holes.

7. The device of claim 6 wherein said member with membrane is sized to be injected percutaneously into a person.

8. An optical test cell to be used with a light measuring instrument comprising:
a fiber optic cord having a plurality of openings into which the substance to be tested may flow.

9. The test cell of claim 8 and further comprising encapsulating material holding said cord having a plurality of channels formed therein leading to said plurality of openings.

10. The test cell of claim 9 wherein said cord is wound in a path in a fashion allowing a single one of said channels to extend across and through said cord at multiple locations along the length thereof.

11. The test cell of claim 10 wherein said openings are holes extending through said cord.

12. The test cell of claim 10 wherein said openings and channels are slots extending through said cord.

13. The test cell of claim 10 and further comprising a frame with upstanding posts around which is wound said cord.

18

14. A remote optical atmosphere monitor cell comprising:

a plate with upstanding posts;

a fiber optic element wound around said posts and extending therebetween in multiple layers; and,

a solidified mass enclosing said element and having a plurality of slots extending across and through said element into which the fluid to be monitored may flow.

15. The monitor cell of claim 14 wherein said posts include means contactable with said element operable to hold said element thereon and limit movement of said layers.

16. The monitor cell of claim 15 wherein said mass is applied to said plate and element in a flowable state and subsequently hardened.

17. A method of detecting a foreign substance in a fluid comprising the steps of:

extending a fiber optic cord through a path;

cutting openings in said cord at multiple locations between its opposite ends;

directing said fluid into said opening;

directing light through said cord; and

comparing said light directed through said cord with said fluid in said openings to a standard.

18. The method of claim 17 wherein said extending step includes winding said cord back and forth in layers; and,

said cutting step includes cutting said cord across said layers.

**Fig.1**

**Fig.2**

**Fig.3**

**Fig.4**

Fig.5

Fig.6